# EUROPÄISCHE PATENTSCHRIFT

(11) **EP 2 193 194 B1**
(45) Veröffentlichungstag und Bekanntmachung des Hinweises auf die Patenterteilung: **30.03.2011**
(21) Anmeldenummer: 08804178.5
(22) Anmeldetag: 15.09.2008
(51) Int. Cl.: C12N 9/02, C12P 7/18

(54) **VERFAHREN ZUR HERSTELLUNG VON 2-METHYL-1,2-DIHYDROXYPROPAN**
METHOD FOR PRODUCING 2-METHYL-1,2-DIHYDROXYPROPANE
PROCÉDÉ DE PRÉPARATION DE 2-MÉTHYL-1,2-DIHYDROXYPROPANE

(30) Priorität: 17.09.2007 DE 102007045093
(43) Veröffentlichungstag der Anmeldung: 09.06.2010
(73) Patentinhaber: Helmholtz-Zentrum für Umweltforschung GmbH - UFZ, 04318 Leipzig (DE)
(72) Erfinder: MÜLLER, Roland H., 04425 Taucha (DE); BREUER, Uta, 04435 Schkeuditz (DE); VON BERGEN, Martin, 04109 Leipzig (DE); BENNDORF, Dirk, 04277 Leipzig (DE); ROHWERDER, Thore, 04299 Leipzig (DE); SCHÄFER, Franziska, 04416 Markkleeberg (DE)
(74) Vertreter: Gulde Hengelhaupt Ziebig & Schneider
(86) Internationale Anmeldenummer: PCT/EP2008/062217
(87) Internationale Veröffentlichungsnummer: WO 2009/037217

(56) Entgegenhaltungen:
- FR-A- 2 888 580
- FERREIRA N L ET AL: "Isolation and characterization of a new Mycobacterium austroafricanum strain, IFP 2015, growing on MTBE" APPLIED MICROBIOLOGY AND BIOTECHNOLOGY, SPRINGER VERLAG, BERLIN, DE, Bd. 70, Nr. 3, 19. Juli 2005 (2005-07-19), Seiten 358-365, XP002380213 ISSN: 0175-7598
- ROHWERDER THORE ET AL: "The alkyl tert-butyl ether intermediate 2-hydroxyisobutyrate is degraded via a novel cobalamin-dependent mutase pathway" APPLIED AND ENVIRONMENTAL MICROBIOLOGY, AMERICAN SOCIETY FOR MICROBIOLOGY, US, Bd. 72, Nr. 6, 1. Juni 2006 (2006-06-01), Seiten 4128-4135, XP002460829 ISSN: 0099-2240
- NICOLAS LOPES FERREIRA ET AL: "Enzymes and genes involved in the aerobic biodegradation of methyl tert-butyl ether (MTBE)" APPLIED MICROBIOLOGY AND BIOTECHNOLOGY, SPRINGER, BERLIN, DE, Bd. 72, Nr. 2, 28. Juni 2006 (2006-06-28), Seiten 252-262, XP019422076 ISSN: 1432-0614
- KANE STACI R ET AL: "Whole-genome analysis of the methyl tert-butyl ether-degrading beta-proteobacterium Methylibium petroleiphilum PM1" JOURNAL OF BACTERIOLOGY, Bd. 189, Nr. 5, März 2007 (2007-03), Seiten 1931-1945, XP002508607 ISSN: 0021-9193 -& DATABASE UniProt 6. März 2007 (2007-03-06), "putative iron sulfur oxidoreductase" XP002508610 gefunden im EBI Database accession no. A2SP35 -& DATABASE UniProt 6. März 2007 (2007-03-06), "Phtalate 4,5 dioxygenase" XP002508611 gefunden im EBI Database accession no. A2SP36
- SCHAEFER F ET AL: "Growth of Aquincola tertiaricarbonis L108 on tert-butyl alcohol leads to the induction of a phthalate dioxygenase-related protein and its associated oxidoreductase subunit" ENGINEERING IN LIFE SCIENCES, Bd. 7, Nr. 5, Oktober 2007 (2007-10), Seiten 512-519, XP002508608 ISSN: 1618-0240
- HRISTOVA KRASSIMIRA R ET AL: "Comparative transcriptome analysis of Methylibium petroleiphilum PM1 exposed to the fuel oxygenates methyl tert-butyl ether and ethanol" APPLIED AND ENVIRONMENTAL MICROBIOLOGY, Bd. 73, Nr. 22, November 2007 (2007-11), Seiten 7347-7357, XP002508609 ISSN: 0099-2240

### SEQUENZPROTOKOLL

<110> Helmhotz-Zentrum für Umweltforschung GmbH - UFZ
<120> Verfahren zur Herstellung von
   2-Methyl-1,2-dihydroxypropan
<130> P508807PC
<140> Neuanmeldung
   <141> 2008-09-12
<150> DE 10 2007 045 093.3
   <151> 2007-09-17
<160> 4
<170> PatentIn Ver. 2.1
<210> 1
   <211> 470
   <212> PRT
   <213> HCM-10
<400> 1
   Met Gly Asn Arg Glu Pro Leu Ala Ala Ala Gly Gln Gly Thr Ala Tyr
    1               5                  10                  15
   Ser Gly Tyr Arg Leu Arg Asp Leu Gln Asn Val Ala Pro Thr Asn Leu
               20                  25                  30
   Glu Ile Leu Arg Thr Gly Pro Gly Thr Pro Met Gly Glu Tyr Met Arg
           35                  40                  45
   Arg Tyr Trp Gln Pro Val Cys Leu Ser Gln Glu Leu Thr Asp Val Pro
       50                  55                  60
   Lys Ala Ile Arg Ile Leu His Glu Asp Leu Val Ala Phe Arg Asp Arg
   65                  70                  75                  80
   Arg Gly Asn Val Gly Val Leu His Arg Lys Cys Ala His Arg Gly Ala
                   85                  90                  95
   Ser Leu Glu Phe Gly Ile Val Gln Glu Arg Gly Ile Arg Cys Cys Tyr
               100                 105                 110
   His Gly Trp His Phe Asp Val Asp Gly Ser Leu Leu Glu Ala Pro Ala
           115                 120                 125
   Glu Pro Pro Asp Thr Lys Leu Lys Glu Thr Val Cys Gln Gly Ala Tyr
       130                 135                 140
   Pro Ala Phe Glu Arg Asn Gly Leu Val Phe Ala Tyr Met Gly Pro Ala
   145                 150                 155                 160
   Asp Arg Arg Pro Glu Phe Pro Val Phe Asp Gly Tyr Val Leu Pro Lys
                   165                 170                 175
   Gly Thr Arg Leu Ile Pro Phe Ser Asn Val Phe Asp Cys Asn Trp Leu
               180                 185                 190
   Gln Val Tyr Glu Asn Gln Ile Asp His Tyr His Thr Ala Leu Leu His
           195                 200                 205
   Asn Asn Met Thr Val Val Gly Val Asp Ala Lys Leu Ala Asp Gly Ala
       210                 215                 220
   Thr Leu Gln Gly Gly Phe Gly Glu Met Pro Ile Ile Asp Trp His Pro
   225                 230                 235                 240
   Thr Asp Asp Asn Asn Gly Met Ile Phe Thr Ala Gly Arg Arg Leu Ser
                   245                 250                 255
   Asp Asp Glu Val Trp Ile Arg Ile Ser Gln Met Gly Leu Pro Asn Trp
               260                 265                 270
   Met Gln Asn Ala Ala Ile Val Ala Ala Ala Pro Gln Arg His Ser Gly
           275                 280                 285
   Pro Ala Met Ser Arg Trp Gln Val Pro Val Asp Asp Glu His Ser Ile
       290                 295                 300
   Ala Phe Gly Trp Arg His Phe Asn Asp Glu Val Asp Pro Glu His Arg
   305                 310                 315                 320
   Gly Arg Glu Glu Glu Cys Gly Val Asp Lys Ile Asp Phe Leu Ile Gly
                   325                 330                 335
   Gln Thr Arg His Arg Pro Tyr Glu Glu Thr Gln Arg Val Pro Gly Asp
               340                 345                 350
   Tyr Glu Ala Ile Val Ser Gln Gly Pro Ile Ala Leu His Gly Leu Glu
           355                 360                 365
   His Pro Gly Arg Ser Asp Val Gly Val Tyr Met Cys Arg Ser Leu Leu
       370                 375                 380
   Arg Asp Ala Val Ala Gly Lys Ala Pro Pro Asp Pro Val Arg Val Lys
   385                 390                 395                 400
   Ala Gly Ser Thr Asp Gly Gln Thr Leu Pro Arg Tyr Ala Ser Asp Ser
                   405                 410                 415
   Arg Leu Arg Ile Arg Arg Arg Pro Ser Arg Glu Ala Asp Ser Asp Val
               420                 425                 430
   Ile Arg Lys Ala Ala His Gln Val Phe Ala Ile Met Lys Glu Cys Asp
           435                 440                 445
   Glu Leu Pro Val Val Gln Arg Arg Pro His Val Leu Arg Arg Leu Asp
       450                 455                 460
   Glu Ile Glu Ala Ser Leu
   465                 470
<210> 2
   <211> 337
   <212> PRT
   <213> HCM-10
<400> 2
   Met Tyr Gln Leu Ser His Thr Gly Lys Tyr Pro Lys Thr Ala Leu Asn
    1               5                  10                  15
   Leu Arg Val Arg Gln Ile Thr Tyr Gln Gly Ile Gly Ile Asn Ala Tyr
               20                  25                  30
   Glu Phe Val Arg Glu Asp Gly Gly Glu Leu Glu Glu Phe Thr Ala Gly
           35                  40                  45
   Ala His Val Asp Leu Tyr Phe Arg Asp Gly Arg Val Arg Gln Tyr Ser
       50                  55                  60
   Leu Cys Asn Asp Pro Ala Glu Arg Arg Arg Tyr Leu Ile Ala Val Leu
   65                  70                  75                  80
   Arg Asp Asp Asn Gly Arg Gly Gly Ser Ile Ala Ile His Glu Arg Val
                   85                  90                  95
   His Thr Gln Arg Leu Val Ala Val Gly His Pro Arg Asn Asn Phe Pro
               100                 105                 110
   Leu Ile Glu Gly Ala Pro His Gln Val Leu Leu Ala Gly Gly Ile Gly
           115                 120                 125
   Ile Thr Pro Leu Lys Ala Met Val His Arg Leu Glu Arg Met Gly Ala
       130                 135                 140
   Asp Tyr Thr Leu His Tyr Cys Ala Lys Ser Ser Ala His Ala Ala Phe
   145                 150                 155                 160
   Gln Glu Glu Leu Ala Pro Met Ala Ala Lys Gly Arg Val Ile Met His
                   165                 170                 175
   Phe Asp Gly Gly Asn Pro Ala Lys Gly Leu Asp Ile Ala Ala Leu Leu
               180                 185                 190
   Arg Arg Tyr Glu Pro Gly Trp Gln Leu Tyr Tyr Cys Gly Pro Pro Gly
           195                 200                 205
   Phe Met Glu Ala Cys Thr Arg Ala Cys Thr Asn Trp Pro Ala Glu Ala
       210                 215                 220
   Val His Phe Glu Tyr Phe Val Gly Ala Pro Val Leu Pro Asp Asp Gly
   225                 230                 235                 240
   Val Pro His Asp Ile Gly Ser Asp Ala Leu Ala Leu Gly Phe Gln Ile
                   245                 250                 255
   Lys Ile Ala Ser Thr Gly Thr Val Leu Thr Val Pro Asn Asp Lys Ser
               260                 265                 270
   Ile Ala Gln Val Leu Gly Glu His Gly Ile Glu Val Pro Thr Ser Cys
           275                 280                 285
   Gln Ser Gly Leu Cys Gly Thr Cys Lys Val Arg Tyr Leu Ala Gly Asp
       290                 295                 300
   Val Glu His Arg Asp Tyr Leu Leu Ser Ala Glu Ala Arg Thr Gln Phe
   305                 310                 315                 320
   Leu Thr Thr Cys Val Ser Arg Ser Lys Gly Ala Thr Leu Val Leu Asp
                   325                 330                 335
   Leu
<210> 3
   <211> 1413
   <212> DNA
   <213> HCM-10
<400> 3
   tcagaggctc gcttcgatct cgtcgaggcg ccgcaggaca tgcggcctgc gctgcacgac 60
   cggcagttcg tcgcactcct tcatgatcgc gaaaacctgg tgcgcggcct tgcggatgac 120
   gtcactgtcc gcttcccggc tcggccggcg gcggatccgc agtcgactgt ccgacgcgta 180
   tcgcggcagc gtttgcccat cggtcgaccc agccttcacg cgcaccgggt cgggcggcgc 240
   cttgccggcc acagcgtcgc gaagcagcga gcgacacatg tacacaccca cgtccgaccg 300
   gccgggatgc tcaagaccgt ggagggctat cggcccctgg ctgacgatgg cttcgtagtc 360
   gcccggaacc cgctgcgtct cttcataagg ccgatgccgg gtctgaccga tcaggaagtc 420
   gatcttgtcg accccgcact cctcttccct tccacggtgc tccgggtcga cctcgtcgtt 480
   gaagtggcgc cagccgaagg cgatcgagtg ctcgtcgtcg accggcacct gccagcgtga 540
   catcgccggg ccggagtgtc gctgcggagc cgccgccacg atggcggcgt tctgcatcca 600
   gttcggcagg cccatctgcg aaattcggat ccagacttcg tcgtccgaca ggcgccggcc 660
   ggcggtgaag atcatgccgt tgttgtcatc ggtcgggtgc cagtcgatga tcggcatttc 720
   gccgaagccc ccctgcagcg tcgcgccgtc ggcgagcttc gcgtccacgc cgacgaccgt 780
   catgttgttg tgcagcagcg cggtgtggta gtggtcgatc tgattttcgt agacctgcag 840
   ccagttgcag tcgaagacat tggagaacgg gatcaaccgc gttcccttcg gcaacacgta 900
   gccgtcgaac accgggaact ctggcctgcg atccgccggc cccatgtagg cgaacaccaa 960
   gccgttgcgc tcgaaggccg gataggcgcc ctggcagacg gtttccttca gcttggtgtc 1020
   ggggggttcc gccggcgcct ccagcaggct gccgtcgacg tcgaagtgcc aaccgtggta 1080
   gcagcaacgg atcccgcgct cctggacgat gccgaactcg agcgaagccc cgcggtgggc 1140
   gcacttgcgg tgcagcacgc cgacgttgcc cctgcgatcc ctgaaagcca ccagatcctc 1200
   gtgcaggatc cgaatcgcct tgggcacgtc ggtcagctcc tgcgacaggc ataccggctg 1260
   ccagtagcgt cgcatgtact cgcccatcgg cgtgccgggg cccgtacgga gaatttccag 1320
   gttcgtgggc gcgacattct gcagatctcg cagccggtac ccgctgtagg ctgtgccctg 1380
   cccggccgcg gccaaaggct ctctgttacc cat                              1413
<210> 4
   <211> 1014
   <212> DNA
   <213> HCM-10
<400> 4
   tcaaagatca aggacgagcg tcgcgccctt cgagcgcgac acgcaggtcg tcaggaactg 60
   cgtgcgtgcc tcggcggaca gcaagtagtc ccgatgctcg acgtcgcccg ccaggtagcg 120
   caccttgcac gttccgcaca ggccgctctg gcacgatgtc ggcacttcga tgccgtgctc 180
   gccaagcacc tgcgcgatcg acttgtcgtt cggtaccgtc aggaccgttc ccgtgctggc 240
   gatcttgatc tggaacccga gcgccagcgc atcgctgccg atgtcgtgag gtactccatc 300
   gtcgggaagc accggcgcgc cgacgaagta ctcgaagtgc accgcctcgg cgggccaatt 360
   ggtgcaggca cgtgtgcagg cctccatgaa cccggggggg ccgcagtagt agagctgcca 420
   acccggctcg taccgccgca gcagcgcagc gatgtccagg cccttggccg gattgccgcc 480
   gtcgaagtgc atgatcacgc gccccttggc ggccatcggc gcgagttcct cctggaacgc 540
   cgcgtgggcg ctcgacttcg cgcagtagtg cagggtgtag tccgcgccca tcctttccaa 600
   ccgatgcacc atggccttca gcggcgtgat gccgatgccg ccggccagca ggacctggtg 660
   gggcgcccct tcaatcagcg ggaagttgtt gcgcgggtgt ccgaccgcga cgagccgttg 720
   cgtgtgcacg cgttcgtgga tcgcgatgga acccccgcgc ccattgtcgt cgcgcagcac 780
   cgcgatcagg tatcgccgac gctcggcggg gtcgttgcac aacgaatact gtcgcacgcg 840
   tccgtcgcgg aagtacagat ccacgtgggc cccggcggtg aactcctcca gttcgccgcc 900
   gtcctcgcgc acgaattcgt aggcgttgat gccgatcccc tggtaggtga tctgccggac 960
   ccgcaggttc agcgccgtct tcgggtactt gccggtgtga ctcaactgat acat       1014

## Beschreibung

Die Erfindung betrifft ein enzymatisches Verfahren zur Herstellung von 2-Methyl-1,2-dihydroxypropan aus Verbindungen, die einen *tert*-Butylrest aufweisen und die unter Verwendung eines Enzymsystems, das die enzymatische Aktivität einer Monooxygenase besitzt, zum Zielprodukt umgewandelt werden. Vorzugsweise betrifft die Erfindung einen biotechnologischen Prozess zur Produktion von 2-Methyl-1,2-dihydroxypropan, wobei Mikroorganismen, die die gewünschte Monooxygenase-Aktivität besitzen oder produzieren, in einem wässrigen System kultiviert werden und die *tert*-Butylverbindungen, wie z.B. den *tert*-Butylrest in Methyl-*tert*-butylether (MTBE), Ethyl-*tert*-butylether (ETBE), *tert*-Amyl-methylether (TAME), *tert-*Butoxymethanol und/oder *tert*-Butylalkohol (TBA) ggf. über Zwischenprodukte zur entsprechenden Zielverbindung umwandeln. Vorzugsweise wird *tert*-Butylalkohol (TBA) als Substrat verwendet.

2-Methyl-1,2-dihydroxypropan (2M12DHP), und daraus abgeleitete Folgeprodukte sind von Interesse für die chemische Industrie. So ist z.B. Propan-1,2-diol eine technisch bedeutende ölige, farblose, fast geruchlose, hygroskopische, viskose Flüssigkeit. Die Synthese von 1,2-Propandiol kann auch mittels mikrobieller Aktivitäten erfolgen. Die Hauptanwendung von Propan-1,2-diol liegt in der Herstellung von Alkyd- und Polyesterharzen (Kunststoffe und Faserglas im Automobil- und Schiffbau und in Konstruktionen, Lackrohstoffe) und als Weichmacher für Vinylharze. Es dient als Starter zur Herstellung von Polyurethanen, als Vernetzer für Polymere und als Reagenz zur Polymermodifikation. Propan-1,2-diol wird in Desinfektionsmitteln, Detergenzien und Reinigungsmitteln genutzt. Die Verbindung wird in Kosmetika als Trägersubstanz in verschiedenen Salben, Cremes und Arzneimitteln genutzt. Propan-1,2-diol (E 1520) ist begrenzt zugelassen als Lebensmittelzusatzstoff und ist zugelassen als Extraktionslösemittel für die Herstellung von Aromen aus natürlichen Aromaträgern.

2-Methyl-1,2-dihydroxypropan (2M12DHP) und daraus abgeleitete Folgeprodukte führen auch zu optisch aktiven Verbindungen (EP1 550 730 A1) oder sind Vorstufen für solche. Die optisch aktiven (*R*)-( -)- und (*S*)-(+)-Formen finden als chiraler Baustein in organischen Synthesen Verwendung und sind Testsubstrate für die Entwickung von Trennmethoden für Enantiomere. Durch Veresterung und/oder Veretherung einer oder beider Hydroxy-Gruppen von Propan-1,2-diol lassen sich zahlreiche als Lösemittel, Weichmacher, Verdickungsmittel oder Emulgatoren verwendbare Produkte gewinnen. Methyl-*tert*.butylether (MTBE) und verwandte Verbindungen wie Ethyl-*tert*.butylether (ETBE) und *tert*.-Amyl-methylether (TAME) werden seit den 80-iger Jahren des vorigen Jahrhunderts als sog. Oxygenate zur Erhöhung der Oktanzahl in Kraftstoffen eingesetzt. Der intensive Umgang mit diesen Verbindungen hat zu einer Belastung der Umwelt geführt. Möglichkeiten für eine Entlastung von Kontaminationen werden wegen seiner Universalität und Versatilität im Allgemeinen im metabolischen Potenzial von Mikroorganismen gesehen. MTBE und verwandte Substanzen erweisen sich allerdings als rekalzitrant. Folgerichtig ist der wachstumsgekoppelte Abbau von MTBE und anderen Oxygenaten trotz intensiver Suche auf wenige Bakterienstämme beschränkt geblieben. Diese sind im Wesentlichen *Methylibium petroleiphilum* PM1, Me*thylibium* sp. R8, *Hydrogenophaga flava* ENV735, *Mycobacterium austroafricanum* IFP2012 und IFP2015und *Variovorax paradoxus* CL-8. Es gibt im Weiteren einige weniger intensive untersuchte Stämme, die metabolische Aktivitäten gegen MTBE zeigen

Der Stoffwechsel von MTBE ist allerdings nur teilweise aufgeklärt. Der Abbau beginnt prinzipiell mit einer Spaltung der Etherbindung, dafür kommt ein Monooxygenase-Mechanismus in Frage. Für *Rhodococcus ruber* IFP2001 bzw. *Aquincola tertiaricarbonis* L108 wird dieser Schritt durch ein P450-abhängiges Enzym katalysiert, codiert durch die Gene *ethABCD.* Das aus der Spaltung resultierende *tert*-Butoxymethanol führt über chemische Dismutation oder eine entspr. Dehydrogenase zu *tert-*Butylalcohol (TBA). Dieses Intermediat findet man häufig in MTBE abbauenden Kulturen, d.h., dass der Schritt in der weiteren Metabolisierung dieser Verbindung unter diesen Umständen geschwindigkeitsbestimmend ist.

Kürzlich konnte in *Mycobacterium austroafricanum* IFP2012 gezeigt werden, dass AlkB, die typische und weit verbreitet n-Alkan Monooxygenase in Gegenwart von TBA indiziert wird (Lopez Ferreira et al. Appl. Microbiol. Biotechnol. 2007, 75, 909-919). AlkB als allgemeine n-Alkan Monooxygenase verfügt bekanntlich über ein weites Substratspektrum. Die Substratspezifität von AlkB-typischen Enzymen ist allerdings variabel. Die Wachstums- bzw. Umsatzraten von Stamm IFP2012 für TBA sind allerdings gering, die maximale spezifische Wachstumsrate µₘₐₓ liegt bei nur 0.039 h⁻¹. Die Tatsache, dass die Umsatzraten so klein sind, spricht dafür, dass es sich um eine unspezifische Reaktion des Enzyms handelt. Für einen anderen grampositiven Stamm konnte ein µₘₐₓ auf TBA von 0.032 h⁻¹ ermittelt werden, zu den beteiligten Enzymen gibt es keine Angaben. Für *Variovorax paradoxus* CL-8 werden ähnlich kleine Raten von µₘₐₓ = 0.042 h⁻¹ erhalten (Zaitsev et al. 2007, Appl. Microbiol. Biotechnol. 74:1092). Auch hier ist das den initialen Schritt katalysierende Enzym unbekannt. Für andere Stämme gibt es bezüglich des beteiligten Enzyms keine und betreffs der realisierten Substratumsätze wenige weitere verwertbare Angaben.

Da TBA in großen Mengen verfügbar ist, besteht das Ziel darin, es als Ausgangssubstanz effizient zu nutzen. Die über AlkB katalysierten Enzymaktivitäten (wie in Stamm IFP2012 gezeigt) sind allerdings zu klein, um Prozesse zur Konversion von TBA mit hinreichend hoher Produktivität zu gewährleisten.

Der Erfindung lag deshalb die Aufgabe zugrunde, nach einem TBA-oxidierenden Enzym zu suchen, das hohe Umsatzraten gewährleistet sowie ein Verfahren zur Herstellung von 2-Methyl-1,2-dihydroxypropan bereitzustellen, das TBA nutzen kann.

Die Aufgabe wird durch ein Verfahren zur enzymatischen Herstellung von 2-Methyl-1,2-dihydroxypropan gelöst, das dadurch gekennzeichnet ist, dass man mindestens eine Verbindung mit einem *tert*-Butylrest in einer wässrigen Reaktionslösung, die ein Enzymsystem aufweist und/oder dieses produziert, das mindestens eine der SEQ ID No. 1 und SEQ ID No. 2 und/oder deren zu mindestens 50% Homologen umfasst und Monooxygenase-Aktivität besitzt, inkubiert und anschließend das entsprechend umgewandelte 2-Methyl-1,2-dihydroxypropan gewinnt, wobei das die Monooxygenase-Aktivität aufweisende Enzymsystem ein isoliertes Enzym, ein isoliertes oligomeres Enzymsystem und/oder ein Mikroorganismus oder ein Rohextrakt davon ist, der das Enzymsystem aufweist bzw. produziert.

Als Verbindungen mit *tert*-Butylrest werden bevorzugt Methyl-*tert*-butylether (MTBE), Ethyl-*tert*-butylether (ETBE), *tert*-Amyl-methylether (TAME), *tert*-Amylalkohol (TAA), *tert*-Butoxymethanol und/oder *tert*-Butylalkohol (TBA) verwendet.

Das erfindungsgemäß eingesetzte Enzymsystem besitzt Monooxygenase-Aktivität und weist das Protein mit der Sequenz SEQ ID No. 1 und das Protein mit der SEQ ID No. 2 auf und/oder eines dieser Proteine in Kombination mit einem jeweiligen zu mindestens 50% Homologen und/oder entsprechende Homologe von Protein 1 und Protein 2, wobei das Protein 1 eine Phthalatdioxygenase darstellt und Mono-Hydroxylase-Aktivität besitzt und das Protein 2 Fe/S-Oxidoreduktase-Aktivität besitzt. Wesentlich ist, dass die Homologen die gleichen Eigenschaften besitzen.

In einer bevorzugten Ausführungsvariante der Erfindung ist das Verfahren ein biotechnologisches Verfahren, wobei Mikroorganismen kultiviert werden, die ein oder mehrere Gene enthalten, die ein oder mehrere Enzyme der SEQ ID No. 1 und 2 und/oder deren zu mindestens 50% Homologen mit Monooxygenase-Aktivität produzieren oder die diese Enzyme aufweisen, wobei sie in wässrigen Systemen die Umwandlung in 2-Methyl-1,2-dihydroxypropan bewirken.

Insbesondere werden Mikroorganismen eingesetzt, die ein Protein codieren, das als oligomeres Enzym zusammengesetzt aus zwei verschiedenen Untereinheiten die unter SEQ. ID Nr. 1 und SEQ ID Nr. 2 angegebenen Aminosäuresequenzen oder deren zu mindestens 50% Homologen aufweisen.

Bevorzugt wird TBA als Substrat eingesetzt direkt oder als Produkt des Abbaus einer der anderen Verbindung mit *tert*-Butylrest. Dabei handelt es sich vorzugsweise um Methyl-*tert*-butylether (MTBE), Ethyl-*tert*-butylether (ETBE), *tert*-Amyl-methylether (TAME), *tert*-Amylalkohol (TAA), oder *tert*-Butoxymethanol, wobei dann in Folge eine Methylgruppe von TBA zur Umwandlung in 2-Methyl-1,2-dihydroxypropan oxidiert wird.

Bevorzugt eingesetzte Mikroorganismen sind Bakterienstamm HCM-10 (DSM 18028), *Ideonella sp.* L108 (DSM 18260) oder *Methylibium sp.* R8 (DSM 19669). Mit diesen Stämmen wurden bevorzugt geeignete biologische Systeme gefunden. Stamm HCM-10 wurde gemäß Budapester Vertrag über die Hinterlegung von Mikroorganismen für die Zwecke von Patentverfahren bei der Deutschen Sammlung von Mikroorganismen und Zellkulturen GmbH, Braunschweig, DE unter der Nr. DSM 18028 am 03.03.2006 hinterlegt; Stamm *Ideonella sp.* L108 wurde am 28.04.2006 bei der Deutschen Stammsammlung für Mikroorganismen und Zellkulturen in Braunschweig, DE unter der Nummer DSM 18260 hinterlegt, und Stamm *Methylibium sp.* R8 wurde am 04.09.2007 bei der Deutschen Stammsammlung für Mikroorganismen und Zellkulturen in Braunschweig, DE unter der Nummer DSM 19669 hinterlegt.

Es wurde überraschend gefunden, dass, wenn insbesondere die genannten Stämme mit einem identischen bzw. ähnlichen Enzym auf TBA oder einem anderen Substrat mit tertiärem Kohlenstoffatom wie z.B. MTBE oder ETBE inbubiert werden, es zu einer Ausscheidung von 2M12DHP aus der Zelle und der Akkumulation im Medium kommt. Die Anwesenheit der oben genannten Substrate führt zur Induktion einer so genannten "TBA-Monooxygenase" für den Fall, dass das betreffende Gen nicht konstitutiv exprimiert wird. Das trifft z.B. für Stamm L108 zu. Der weitere Verlauf der Inkubation des Enzyms, das nach wie vor als ganze Zelle, als Zellextrakt oder als angereichertes oder gereinigtes Enzymsystem vorliegen kann, so gesteuert wird, dass die Umsetzung des Substrates, im speziellen Fall von TBA unvollständig in dem Sinne ist, dass das gewünschte Zielsubstrat, das 2M12DHP, in der Inkubationsflüssigkeit anreichert wird und in geeigneter Weise gewonnen werden kann. Zur Erreichung dieses Ziel bzw. zur Steigerung der Ausbeute können auch Enzyme gehemmt oder ausgeschaltet werden, die 2M12DHP weiter umsetzen können. Beispielsweise sei das als Sulfolactat-Dehydrogenase [L-sulfolactate dehydrogenase (EC:1.1.1.-)] ausgewiesene Protein YP_001023561 (Mpe_B0558), das zu 100% identisch zu einem PM1 annotierten Protein ist, genannt.

Der Inkubationsansatz kann weitere Aktivitätskomponenten enthalten. Insbesondere ist bei Einsatz von subzellulären Systemen die Regenerationskapazität für Reduktionsäquivalente zu berücksichtigen, die für die Monooxygenase-Funktion verbraucht werden und über geeignete Systeme und aus geeigneten Quellen regeneriert werden müssen.

Die bevorzugt genannten Stämme sind demzufolge eine Quelle für ein geeignetes Enzymsystem zur Konversion von TBA in 2M12DHP. Z.B. der Stamm DSM 18028 wächst mit Raten von µₘₐₓ = 0.1 h⁻¹ auf TBA. Diese Rate führt über die Ausbeute auf TBA von ca. 35 g/mol zu TBA Umsatzraten, die sich auf bis zu 210 mmol/h*g Biomasse belaufen.

Die Reaktionsbedingungen (pH-Wert, Ionenkonzentration, Sauerstoff/ Kohlendioxidbedarf, Spurenelemente, Temperaturen und ähnliches) werden dabei selbstverständlich so gewählt, dass die Mikroorganismen zu einer optimalen Umsetzung von TBA zu 2-Methyl-1,2-dihydroxypropan befähigt sind. Stamm DSM 18028 oder DSM 18260 zeigen z.B. vorzugsweise Aktivitäten im pH-Bereich von 5,0 bis 9,0, bevorzugt zwischen 5,5 und 8,5. Der Temperaturbereich für die Aktivität dieser Stämme liegt bevorzugt zwischen 5°C und 40°C, besonders bevorzugt zwischen 22°C und 37°C und ganz besonders bevorzugt bei 30°C. Unter diesen Verfahrensbedingungen kann das Enzymsystem im natürlichen Mikro-Milieu, also innerhalb der Zelle, eine höhere Stabilität und Effektivität aufweisen als das isolierte Enzym. Darüber hinaus kann unter geeigneten Bedingungen eine Zellvermehrung und somit eine Erhöhung der EnzymKonzentration möglich sein. Somit bedeutet die enzymatische Umsetzung mittels Mikroorganismen ggf. einen bedeutenden Vorteil, was Zuverlässigkeit, Automatisierung und Einfachheit sowie Qualität und Ausbeute des Endproduktes des Verfahrens angeht.

Für die erfindungsgemäße enzymatische Umsetzung ist es auch möglich, das Enzymsystem in gereinigter, angereicherter und/oder isolierter Form in die Reaktionslösung einzubringen, wobei die Enzyme z.B. natürlichen Ursprungs sein können. Selbstverständlich können die Enzyme auch rekombinant hergestellte Enzyme aus einem gentechnisch veränderten Organismus sein.

Die Enzyme werden im erfindungsgemäßen Verfahren im Sinne der Erfindung als Katalysatoren sowohl in Form von intakten mikrobiellen Zellen als auch in Form von permeabilisierten mikrobiellen Zellen eingesetzt. Weitere Einsatzmöglichkeiten bestehen in Form von Komponenten (eine oder mehrere) aus mikrobiellen Zellextrakten, aber auch in partiell gereinigter oder gereinigter Form. Gegebenenfalls werden weitere Enzyme, z.B. eine Formiat-Dehydrogenase erfindungsgemäß eingesetzt. Die enzymatischen Katalysatoren können immobilisiert sein oder an ein gelöstes oder ungelöstes Trägermaterial angeheftet sein.

In einer bevorzugten Ausführungsvariante werden bestimmte Zellkompartimente oder Teile davon voneinander getrennt oder vereinigt, das heißt, Kohlenhydratstrukturen, Lipide oder Proteine und/oder Peptide sowie Nukleinsäuren, die in der Lage sind, die Monooxygenase-Aktivität aufweisende Einheit positiv oder negativ zu beeinflussen, können kombiniert oder getrennt werden. Um eine solche Beeinflussung bewusst zu nutzen, werden aus den Mikroorganismen z.B. fachgemäß Rohextrakte hergestellt, welche ggf. zentrifugiert werden um eine erfindungsgemäße Umsetzung mit dem Sediment oder dem Überstand durchführen zu können.

Unter Verwendung dieser bevorzugten biologischen Systeme konnte eine besonders gute Ausbeute an 2-Methyl-1,2-propandiol erzielt werden. Jedoch ist die enzymatische Umsetzung durch Mikroorganismen keinesfalls auf diesen Stamm beschränkt. Sämtliche Organismen, die in der Lage sind, TBA in 2-Methyl-1,2-propandiol umzuwandeln, können erfindungsgemäß eingesetzt werden.

Dabei kann es sich um Mikroorganismen handeln, die zum einen über das gleiche Gen bzw. Genprodukt verfügen oder zum anderen ein analoges Gen besitzen, welches zu Genprodukten mit ähnlicher oder analoger Aktivität führt. Ebenso schließt die Erfindung transformierte Systeme, die eine oder eine ähnliche Monooxygenase-Aktivität wie die Stämme HCM-10, L108 oder R8 oder die anderer Herkunft besitzen, mit ein. Dazu können Mutanten, gentechnisch geänderte sowie isolierte Abwandlungen der Mikroorganismen zählen, z.B. Organismen, die aufgrund des Einführens einer Monooxygenase-codierenden Nukleotidsequenz die gewünschte Monooxygenase-Aktivität aufweisen.

Die erfindungsgemäß verwendeten Stämme produzieren bevorzugt die Proteine mit den Sequenzen SEQ ID NO: 1 und/oder SEQ ID NO: 2 bzw. umfassen die Nukleinsäuresequenzen SEQ ID NO: 3 und/oder SEQ ID NO: 4. Im Sinne der Erfindung können die genannten Proteine auch in angereicherter, isolierter oder synthetisch hergestellter Form eingesetzt werden.

In einer weiteren bevorzugten Ausführungsvariante der Erfindung werden die Enzym-Katalysatoren, insbesondere Mikroorganismen, Rohextrakte, Teile davon und/oder die angereicherten oder isolierten Enzyme immobilisiert eingesetzt. Durch die Immobilisierung werden Enzyme, Zellorganellen und Zellen in einen unlöslichen und reaktionsraumbegrenzten Zustand versetzt. So können sie z.B. in einer Polymer-Matrix immobilisiert werden (z. B. Alginat-, Polyvinylalkohol- oder Polyacrylamid-Gele). Eine Immobilisierung kann auch auf gelösten oder ungelösten Trägermaterialien (z. B. Celit) zur Erleichterung der Katalysator-Wiedergewinnung und -benutzung erfolgen. Methoden zur Zell-Immobilisierung in einer Polymer-Matrix oder auf einem gelösten oder ungelösten Träger sind dem Fachmann bekannt und bereits ausführlich beschrieben worden. Die Enzymaktivitäten können ebenfalls aus den mikrobiellen Zellen isoliert werden. Diese können dann direkt als Katalysator oder in einer Polymer-Matrix oder auf einem gelösten oder ungelösten Träger immobilisiert eingesetzt werden. Die dazu nötigen Methoden sind dem Fachmann bekannt und beschrieben z.B. in Methods in Biotechnology, Bd. 1: Immobilization of enzymes and cells, Herausgeber: G. F. Bickerstaff, Humana Press, Totowa, New Jersey, 1997.

Die Umwandlung von TBA in 2-Methyl-1,2-dihydroxypropan erfolgt bevorzugt im Rahmen eines kontinuierlichen Verfahrens, welches in einem durchströmten Reaktor durchgeführt werden kann, in dem mikrobielles Wachstum und somit die Produktbildung stattfindet. Unter einem kontinuierlichen Verfahren kann jedoch auch jedes System wachsender Zellen und katalysierender Enzyme verstanden werden, dem einerseits Nährlösung zugeführt wird und aus dem andererseits Kulturlösung, einschließlich enzymatisch gebildeten Zielprodukts abgezogen wird. Erfindungsgemäß kann das Verfahren auch als semikontinuierliches oder Batch-Verfahren durchgeführt werden.

In einer ganz besonderen Ausführung der Erfindung wird zur Kultivierung TBA als einziges Kohlenstoffsubstrat in einem Basalmedium eingesetzt.

In Rahmen der Erfindung werden die einen *tert*-Butylrest tragenden Substrate als Konversionssubstrat bezeichnet, weitere Substrate, die gegebenenfalls zum Einsatz kommen, dienen als Wachstumssubstrate oder als auxiliare Substrate.

So kann die Kultivierung der Mikroorganismen in Gegenwart von weiteren Zusatzstoffen erfolgen. Die Kultivierung erfolgt z.B. in Gegenwart von einem Zucker, einer organischen Säure, einem Alkohol oder einem Kohlenwasserstoff als einziger Kohlenstoff- und Energiequelle in einem Basalmedium oder auch in Gegenwart eines Gemisches von unterschiedlichen Substraten, bestehend aus Zuckern, organischen Säuren, Alkoholen und/oder Kohlenwasserstoffen in variabler Zusammensetzung. Dabei kann das Verfahren so durchgeführt werden, dass nach Kultivierung auf einem Zucker, einer organische Säure, einem Alkohol oder einem Kohlenwasserstoff als einziger Kohlenstoff- und Energiequelle oder einem Gemisch von diesen Substanzen in einem Basalmedium zur Induktion der erfindungsgemäß verwendeten Monooxygenase die *tert-*Butylverbindung, vorzugsweise TBA oder eine andere, ein tertiäres C-Atom tragende Verbindung oder ein Gemisch von solchen Verbindungen zugesetzt wird, wobei nach der Induktion die Umsetzung der ein tertiäres C-Atom tragenden Verbindung, vornehmlich TBA, in 2-Methyl-1,2-dihydroxypropan erfolgt.

In einer weiteren Ausführungsvariante der Erfindung werden die ein tertiäres Kohlenstoff-Atom tragenden Substrate oder derartige Substratgemische vorzugsweise simultan mit dem oder den anderen weiteren Substraten oder Substratgemischen kultiviert.

Die Kultivierung erfolgt diskontinuierlich, semikontinuierlich oder kontinuierlich. Bevorzugt wird das Mischungsverhältnis zwischen Wachstumssubstrat und Konversionssubstrat variiert. Bevorzugt wird ein definiertes Mischungsverhältnis zwischen Wachstumssubstrat und Konversionssubstrat eingestellt, welches eine hohe Produktivität für die Bildung von 2-Methyl-1,2-dihydroxypropan gewährleistet. Das Mischungsverhältnis ist abhängig von Reduktionsgrad des Wachstumssubstrates und der Effizienz der E-nergiewandlung, d.h. auch der Freisetzung von Reduktionsäquivalenten [H₂] im Stoffwechsel, wobei [H₂] bevorzugt NADH + H⁺ und/oder NADPH + H⁺ darstellen. Die Effizienz und das daraus abgeleitete Mischungsverhältnis wird deshalb pro C-Atom als [H₂]pro C-mol angegeben und eingesetzt und errechnet sich aus den allgemein bekannten Stoffwechselwegen für die einzelnen Wachstumssubstrate (Babel und Müller 1985; J. Gen Microbiol 131:39), wobei pro C-mol mindestens 1 Reduktionsäquivalent freigesetzt wird. Bevorzugt liegt das Verhältnis für [H₂]pro C zu einem Molekül des Konversionssubstrates zwischen 1 zu 1 bis 1 zu 0,01, besonders bevorzugt zwischen 1 zu 0,5 bis 1 zu 0,1.

In einer Ausführungsvariante wird ETBE als Ausgangssubstrat eingesetzt. Durch Spaltung der Etherbindung wird Acetat freigesetzt, aus dessen weiterer Metabolisierung Reduktionsäquivalente hervorgehen, die als Cosubstrat für die TBA Monooxygenase genutzt werden können und zur Umsetzung von TBA in 2-Methyl-1,2-dihydroxypropan führen.

In einer weiteren Ausführungsvariante wird das Verfahren mit stationären Kulturen (nicht nachwachsenden Zellen) ohne Wachstumssubstrate durchgeführt. Zur Regenerierung von Reduktionsäquivalenten kann gegebenenfalls ein auxiliares Substrat zugesetzt werden, wobei vorzugsweise zur Regenerierung von Reduktionsäquivalenten Ameisensäure oder deren Salze zugesetzt werden.

Zur Verwertung der auxiliaren Substrate werden gegebenenfalls entsprechende Gene kloniert, die exprimiert werden und zum Umsatz dieser Substrate über die entsprechenden Enzyme führen. So besitzt z.B. Stamm DSM18028 oder DSM 18260 keine wesentliche Formiat Dehydrogenase Aktivität, soll dieses Substrat eingesetzt werden, kann das Gen problemlos kloniert werden. Formiat ist deshalb zu bevorzugen, da nach der Konversion nur CO₂ entsteht, welches eine Produktreinheit nicht beeinträchtigt.

Das ins Medium ausgeschiedene 2-Methyl-1,2-dihydroxypropan wird in geeigneter Weise gewonnen, z.B. über fraktionierte Destillation.

Das Verfahren kann aerob, vorzugsweise beim Einsatz ganzer Zellen, durchgeführt werden oder auch micro-aerob, z.B. unter Stickstoffbegasung, vorzugsweise wenn Extrakte oder gereinigte Enzyme verwendet werden, wobei in diesem Falle die Sauerstoffbereitstellung im Sinne einer Substratzufuhr gesteuert wird.

Es hat sich gezeigt, dass das erfindungsgemäß eingesetzte Enzymsystem bevorzugt ein heterodimeres Protein darstellt, welches die unter SEQ ID No. 1 und SEQ ID Nr. 2 beschriebenen Untereinheiten umfasst bzw. daraus besteht und so eine hervorragende Enzymaktivität besitzt. Es hat sich gezeigt, dass Enzyme/Enzymsysteme mit mindestens 50% Sequenzhomologie eine ebenfalls hervorragende gewünschte Enzymaktivität besitzen. Die verwendeten Proteine können aus natürlichen Quellen isoliert werden, vorzugsweise aus DSM 18028, DSM 18260 oder DSM 19669 oder sie können nach bekannten Verfahren synthetisiert werden.

Homologie bedeutet dabei eine Sequenzidentität von mindestens 40%, insbesondere eine Identität von mindestens 60%, vorzugsweise über 80% und besonders bevorzugt über 90%, 95%, 97% oder 99% auf Nukleinsäureebene. Dabei weisen die codierten Enzyme eine Sequenzidentität zu den angegebenen Aminosäuresequenzen von mindestens 50%, vorzugsweise von mindestens 60% bzw. 80%, besonders bevorzugt von mindestens 95%, ganz besonders bevorzugt mindestens 97% oder 99% auf Aminosäureebene auf. Die Abweichungen können dabei durch Deletion, Substitution, Insertion oder Rekombination entstanden sein. Es kann sich dabei um natürlich auftretende Variationen handeln, beispielsweise um Sequenzen aus anderen Organismen, oder um Mutationen, wobei diese Mutationen auf natürliche Weise auftreten können oder durch gezielte Mutagenese (UV-Strahlen, Röntgenstrahlen, chemische Mittel oder weitere). Ferner kann es sich bei den Varianten um synthetisch hergestellte Sequenzen handeln. Diese Varianten weisen bestimmte gemeinsame Charakteristika auf, wie z.B. Enzymaktivität, aktive Enzymkonzentration, Untereinheiten, funktionelle Gruppen, konservierte Sequenzen bzw. Sequenzmotive, immunologische Reaktivität, Konformation und/oder physikalische Eigenschaften, wie das Laufverhalten in Gelelektrophorese, chromatographisches Verhalten, Löslichkeit, Sedimentationskoeffizienten, pH-Wert-Optimum, Temperatur-Optimum, spektroskopische Eigenschaften, Stabilität und/oder andere.

### Abb. 1

SEQ ID NO: 1 zeigt die Phthalatdioxygenase mit Hydroxylase-Aktivität mit 470 Aminosäuren (große Untereinheit des Enzymsystem mit der enzymatischen Aktivität einer Monooxygenase) aus DSM 18028.

SEQ ID NO: 2 zeigt die 337 AS umfassende Sequenz mit Fe/S-Oxidoreduktase-Aktivität (kleine Untereinheit des Enzymsystem mit der enzymatischen Aktivität einer Monooxygenase) aus DSM 18028.

### Abb. 2

SEQ ID NO: 3 zeigt 1413 bp der codierenden Nukleotidsequenz für die Phthalatdioxygenase aus DSM 18028.

### Abb. 3

SEQ ID NO: 4 zeigt 1014 bp der codierenden Nukleotidsequenz für die Fe/S-Oxidoreduktase aus DSM 18028.

### Ausführungsbeispiel

### Beispiel 1

Der Stamm DSM 18028 wurde auf Mineralsalzmedium (MSM) in Gegenwart einer Vitaminmischung kultiviert. Das Medium enthielt (in mg/l): NH₄Cl, 350; KH₂PO₄, 340; K₂HPO₄, 485; CaCl₂ * 6 H₂O, 27; MgSO₄ * 7 H₂O, 71.2, und 1 ml/l Spurensalz-Stammlösung. Die Spurensalz-Stammlösung enthielt (in g/l): FeSO₄ * 7 H₂O, 4.98; Cu-SO₄ * 5 H₂O, 0.785; CoCl₂, 5; MnSO₄ * 4 H₂O, 0.81; ZnSO₄ * 7 H₂O, 0.44; Na₂MoO₄ * 2 H₂O, 0.25. Die Konzentration der Vitamin im Medium betrug (in µg/l): Biotin, 20; Folsäure, 20; Pyridoxin-HCl, 100; Thiamin-HCl, 50; Riboflavin, 50; Nikotinsäure, 50; DL-Ca-Pantothenat, 50; p-Aminobenzoesäure, 50; Liponsäure, 50, und Cobalamin, 50. Als Wachstumssubstrat diente Succinat in einer Konzentration von 3 g/l. Die Kultivierung erfolgte kontinuierlich oder diskontinuierlich. Bei kontinuierlicher Kultivierung wurde zusätzlich *tert*.-Butylalkohol (TBA) in einer Konzentration von 0,2 g/l zugeführt. Bei diskontinuierlicher Kultivierung wurde die Kultur unmittelbar weiter verwendet bzw. der Kultur im Anschluss an das Wachstum auf Succinat zur Induktion der Monooxygenase-Aktivität TBA in einer Konzentration von 0,2 g/l zugesetzt und die Kultur für weitere 5 h inkubiert.

Nach kontinuierlicher bzw. diskontinuierlicher Kultivierung wurden die Zellen abgetrennt, gewaschen und mit Phosphatpuffer (50 mM, pH 7,0) auf eine Biomassekonzentration von 0,5 g/l eingestellt. Dieser Suspension wurde *tert*.-Butylalkohol (TBA) in einer Konzentration von 10 mM zugesetzt und der Ansatz in einem gasdicht verschlossenen Gefäß bei 30°C inkubiert. Das Verhältnis von Flüssig- zu Gasvolumen lag bei 4 zu 1. Als Ausgangsgas diente Raumluft. Nach 24 h wurden unter diesen Bedingungen und unter Einsatz diskontinuierlich kultivierter Zellen 5,2 mM 2-Methyl-1,2-dihydroxypropan gebildet, bei Einsatz kontinuierlich kultivierter und induzierter Zellen wurden nach 24 h Inkubationszeit 5,5 mM 2-Methyl-1,2-dihydroxypropan erhalten, bei Einsatz nicht-induzierter Zellen lag die Konzentration an 2-Methyl-1,2-dihydroxypropan nach 24 h bei 3,9 mM.

### Beispiel 2

Der Stamm DSM 18028 wurde auf einem Medium wie in Beispiel 1 angegeben vorkultiviert, allerdings in Gegenwart von 760 mg/l NH₄Cl. Nach der Kultivierung wurde die Suspension für eine nachfolgende Produktbildungsphase wie oben angeführt in Phosphatpuffer entsprechend aufbereitet.

Dieser Suspension wurde *tert*.-Butylalkohol (TBA) in einer Konzentration von 10 mM sowie 1 g/l Succinat zugesetzt und der Ansatz in einem gasdicht verschlossenen Gefäß bei 30°C unter den in Beispiel 1 angegebenen Bedingungen inkubiert. Nach 24 h wurden unter diesen Bedingungen 6,1 mM 2-Methyl-1,2-dihydroxypropan gebildet.

### Beispiel 3

Der Stamm DSM 18028 wurde auf einem Medium wie in Beispiel 2 angegeben vorkultiviert. Nach der Kultivierung wurde die Suspension für eine nachfolgende Produktbildungsphase wie oben angeführt in Phosphatpuffer entsprechend aufbereitet.

Dieser Suspension wurde Methyl-*tert*.-Butylether (MTBE) in einer Konzentration von 10 mM sowie 1 g/l Succinat zugesetzt und der Ansatz in einem gasdicht verschlossenen Gefäß bei 30°C unter den in Beispiel 1 angegebenen Bedingungen inkubiert. Nach 24 h wurden unter diesen Bedingungen 2,4 mM 2-Methyl-1,2-dihydroxypropan gebildet.

### Beispiel 4

Der Stamm DSM 18028 wurde auf einem Medium wie in Beispiel 2 angegeben vorkultiviert. Nach der Kultivierung wurde die Suspension für eine nachfolgende Produktbildungsphase wie oben angeführt in Phosphatpuffer entsprechend aufbereitet.

Dieser Suspension wurde Ethyl-*tert*.-Butylether (ETBE) in einer Konzentration von 10 mM sowie 1 g/l Succinat zugesetzt und der Ansatz in einem gasdicht verschlossenen Gefäß bei 30°C unter den in Beispiel 1 angegebenen Bedingungen inkubiert. Nach 24 h wurden unter diesen Bedingungen 2,7 mM 2-Methyl-1,2-dihydroxypropan gebildet.

### Beispiel 5

Der Stamm DSM 19669 wurde auf einem Medium wie in Beispiel 2 angegeben vorkultiviert. Nach der Kultivierung wurde die Suspension für eine nachfolgende Produktbildungsphase wie oben angeführt in Phosphatpuffer entsprechend aufbereitet.

Dieser Suspension wurde *tert*.-Butylalkohol (TBA) in einer Konzentration von 10 mM sowie 1 g/l Succinat zugesetzt und der Ansatz in einem gasdicht verschlossenen Gefäß bei 30°C unter den in Beispiel 1 angegebenen Bedingungen inkubiert. Nach 24 h wurden unter diesen Bedingungen 4,1 mM 2-Methyl-1,2-dihydroxypropan gebildet.

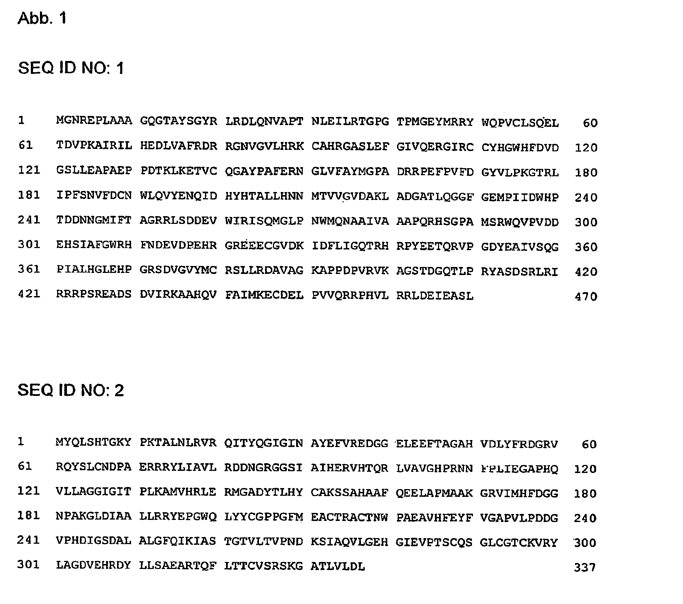

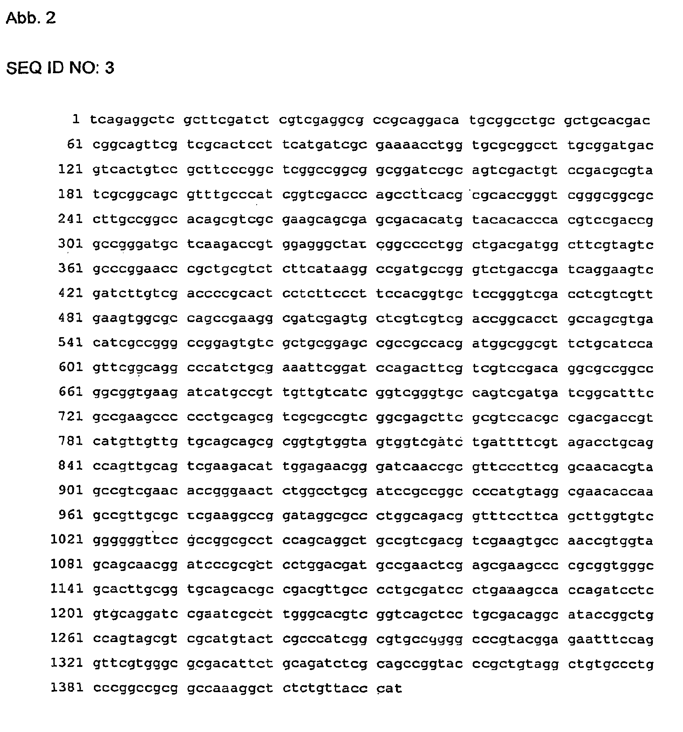

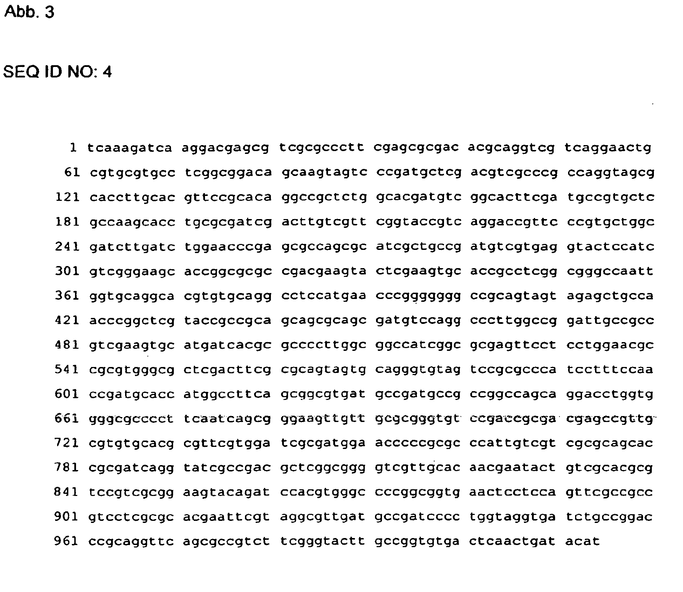

## Patentansprüche

1. Verfahren zur enzymatischen Herstellung von 2-Methyl-1,2-dihydroxypropan **dadurch gekennzeichnet, dass** man mindestens eine Verbindung mit einem *tert*-Butylrest in einer wässrigen Reaktionslösung, die ein Enzymsystem aufweist und/oder dieses produziert, das mindestens eine der SEQ ID No. 1 und SEQ ID No. 2 und/oder deren zu mindestens 50% Homologen umfasst und Monooxygenase-Aktivität besitzt, inkubiert und anschließend das entsprechend umgewandelte 2-Methyl-1,2-dihydroxypropan gewinnt, wobei das die Monooxygenase-Aktivität aufweisende Enzymsystem ein isoliertes Enzym, ein isoliertes oligomeres Enzymsystem und/oder ein Mikroorganismus oder ein Rohextrakt davon ist, der das Enzymsystem aufweist bzw. produziert.

2. Verfahren nach Anspruch 1, **dadurch gekennzeichnet, dass** als Verbindungen mit *tert*-Butylrest Methyl-*tert*-butylether (MTBE), Ethyl-*tert*-butylether (ETBE), *tert*-Amyl-methylether (TAME), *tert*-Amylalkohol (TAA), *tert*-Butoxymethanol und/oder *tert*-Butylalkohol (TBA) verwendet werden.

3. Verfahren nach Anspruch 1 oder 2, **dadurch gekennzeichnet, dass** das Enzymsystem mit Monooxygenase-Aktivität das Protein mit der Sequenz SEQ ID No. 1 und das Protein mit der SEQ ID No. 2 aufweist, und/oder in Kombination mit ihren jeweiligen zu mindestens 50% Homologen, wobei das Protein SEQ ID No. 1 eine Phthalatdioxygenase darstellt und MonoHydroxylase-Aktivität aufweist und das Protein mit SEQ ID No. 2 Fe/S-Oxidoreduktase-Aktivität besitzt, und die Homologen die gleichen Eigenschaften besitzen.

4. Verfahren nach einem der Ansprüche 1 bis 3, **dadurch gekennzeichnet, dass** man Mikroorganismen kultiviert, die ein oder mehrere Gene enthalten, die ein oder mehrere Enzyme der SEQ ID No. 1 und 2 und/oder deren zu mindestens 50% Homologen mit Monooxygenase-Aktivität produzieren oder die diese Enzyme aufweisen, wobei sie in wässrigen Systemen die Umwandlung von der Verbindung(en) mit *tert*-Butylrest in 2-Methyl-1,2-dihydroxypropan bewirken.

5. Verfahren nach einem der Ansprüche 1 bis 4, **dadurch gekennzeichnet, dass** ein Mikroorganismus eingesetzt wird, der ein Protein codiert, das als oligomeres Enzym zusammengesetzt aus zwei verschiedenen Untereinheiten die unter SEQ. ID Nr. 1 und SEQ ID Nr. 2 angegebenen Aminosäuresequenzen aufweist oder der als prokaryotischer Wirt fungiert, in dem das Gen oder die Gene exprimiert werden, die die unter SEQ. ID Nr. 3 und/oder SEQ ID Nr. 4 angegebene Nucleotidsequenz aufweisen.

6. Verfahren nach einem der Ansprüche 1 bis 5, **dadurch gekennzeichnet, dass** TBA als Substrat eingesetzt oder aus einer der anderen Verbindungen von Anspruch 2 gewonnen oder hergestellt wird, wobei eine Methylgruppe von TBA zur Umwandlung in 2-Methyl-1,2-dihydroxypropan oxidiert wird.

7. Verfahren nach einem der Ansprüche 1 bis 6, **dadurch gekennzeichnet, dass** der Mikroorganismus ausgewählt ist aus dem Bakterienstamm HCM-10 (DSM 18028), *Ideonella sp.* L108 (DSM18260) oder *Methylibium sp.* R8 (DSM 19669).

8. Verfahren nach einem der Ansprüche 1 bis 7, **dadurch gekennzeichnet, dass** ganze Zellen der Mikroorganismen, unverändert, permeabilisiert oder trägerfixiert eingesetzt werden.

9. Verfahren nach einem der Ansprüche 1 bis 8, **dadurch gekennzeichnet, dass** Zellextrakte und/oder eine Monooxygenase bzw. ein Monoxygenasesystem nach teilweiser oder vollständiger Isolierung aus den Mikroorganismen, ggf. in gereinigter Form, eingesetzt werden, vorzugsweise zellfreie Rohextrakte der Mikroorganismen.

10. Verfahren nach einem der Ansprüche 1 bis 9, **dadurch gekennzeichnet, dass** zur Kultivierung TBA als einzige Kohlenstoff- und Energiequelle in einem Basalmedium eingesetzt wird.

11. Verfahren nach einem der Ansprüche 1 bis 10, **dadurch gekennzeichnet, dass** zur Kultivierung Wachstumssubstrate zugesetzt werden, vorzugsweise ein Zucker, eine organische Säure, ein Alkohol oder ein Kohlenwasserstoff als einzige oder als zusätzliche Kohlenstoff- und Energiequelle in einem Basalmedium oder ein Gemisch von unterschiedlichen Substraten, bestehend aus Zuckern, organischen Säuren, Alkoholen und/oder Kohlenwasserstoffen.

12. Verfahren nach einem der Ansprüche 1 bis 11, **dadurch gekennzeichnet, dass** die Kultivierung auf einem Wachstumssubstrat bzw. -gemisch vor Zugabe der *tert*-Butylverbindung oder simultan mit dieser zur Induktion der TBA Monooxygenase durchgeführt wird, wobei nach der Induktion die Umsetzung der das tertiäre C-Atom tragenden Verbindung in 2-Methyl-1,2-dihydroxypropan erfolgt.

13. Verfahren nach einem der Ansprüche 1 bis 12, **dadurch gekennzeichnet, dass** das Mischungsverhältnis zwischen Wachstumssubstrat und Substrat mit tert. Butylverbindung variiert wird, wobei vorzugsweise ein Verhältnis von [H₂]pro C-mol und einem Molekül des Konversionssubstrates zwischen 1 zu 1 bis 1 zu 0,01, besonders bevorzugt zwischen 1 zu 0,5 bis 1 zu 0,1 eingesetzt wird, wobei [H₂]pro C-mol dem pro C-mol des Wachstumssubstrates über dem Stoffwechsel verfügbar gemachten Reduktionsäquivalenten entspricht und der Quotient [H₂]pro C-mol mindestens 1 beträgt.

14. Verfahren nach einem der Ansprüche 1 bis 12, **dadurch gekennzeichnet, dass** als Ausgangssubstrat ETBE eingesetzt wird, wobei das nach Spaltung der Etherbrücke freigesetzte Acetat und seine durch weitere Metabolisierung freigesetzten Reduktionsäquivalente zur Oxidation von TBA in Methyl-1,2-dihydroxypropan genutzt werden oder dass die weitere Umsetzung von 2-Methyl-1,2-dihydroxypropan eingeschränkt bzw. unterbunden wird.

15. Verfahren nach einem der Ansprüche 1 bis 10, **dadurch gekennzeichnet, dass** zur Regenerierung von Reduktionsäquivalenten ein auxiliares Substrat zugesetzt wird, vorzugsweise Ameisensäure oder deren Salze.

## Claims

1. Method for the enzymatic production of 2-methyl-1,2-dihydroxypropane, **characterized in that** at least one compound with a tert-butyl radical is incubated in an aqueous reaction solution which has and/or produces an enzyme system which comprises at least one of the SEQ ID No. 1 and SEQ ID No. 2 and/or their at least 50% homologues and has monooxygenase activity, and then the correspondingly converted 2-methyl-1,2-dihydroxy-propane is obtained, where the enzyme system having the monooxygenase activity is an isolated enzyme, an isolated oligomeric enzyme system and/or a microorganism or a crude extract thereof which has and/or produces the enzyme system.

2. Method according to Claim 1, **characterized in that** methyl tert-butyl ether (MTBE), ethyl tert-butyl ether (ETBE), tert-amyl methyl ether (TAME), tert-amyl alcohol (TAA), tert-butoxymethanol and/or tert-butyl alcohol (TBA) are used as compounds with tert-butyl radical.

3. Method according to Claim 1 or 2, **characterized in that** the enzyme system with monooxygenase activity has the protein with the sequence SEQ ID No. 1 and the protein with the SEQ ID No. 2, and/or in combination with their respective at least 50% homologues, where the protein SEQ ID No. 1 is a phthalate dioxygenase and has monohydroxylase activity and the protein with SEQ ID No. 2 has Fe/S oxidoreductase activity, and the homologues have the same properties.

4. Method according to one of Claims 1 to 3, **characterized in that** microorganisms are cultivated which comprise one or more genes which produce one or more enzymes of the SEQ ID No. 1 and 2 and/or their at least 50% homologues with monooxygenase activity or which have these enzymes, where, in aqueous systems, they bring about the conversion of the compound(s) with tert-butyl radical to 2-methyl-1,2-dihydroxypropane.

5. Method according to one of Claims 1 to 4, **characterized in that** a microorganism is used which codes a protein which has, as oligomeric enzyme composed of two different subunits, the amino acid sequences given under SEQ ID No. 1 and SEQ ID No. 2 or which functions as prokaryotic host in which the gene or the genes are expressed which have the nucleotide sequence given under SEQ ID No. 3 and/or SEQ ID No. 4.

6. Method according to one of Claims 1 to 5, **characterized in that** TBA is used as substrate or is obtained or produced from one of the other compounds from Claim 2, where one methyl group of TBA is oxidized for the conversion to 2-methyl-1,2-dihydroxypropane.

7. Method according to one of Claims 1 to 6, **characterized in that** the microorganism is selected from the bacteria strain HCM-10 (DSM 18028), *Ideonella sp*. L108 (DSM18260) or *Methylibium sp.* R8 (DSM 19669).

8. Method according to one of Claims 1 to 7, **characterized in that** whole cells of the microorganisms are used unchanged, permeabilized or carrier-fixed.

9. Method according to one of Claims 1 to 8, **characterized in that** cell extracts and/or a monooxygenase or a monooxygenase system are used following partial or complete isolation from the microorganisms, optionally in purified form, preferably cell-free crude extracts of the microorganisms.

10. Method according to one of Claims 1 to 9, **characterized in that**, for the cultivation, TBA is used as the sole carbon and energy source in a basal medium.

11. Method according to one of Claims 1 to 10, **characterized in that**, for the cultivation, growth substrates are added, preferably a sugar, an organic acid, an alcohol or a hydrocarbon as the sole or as an additional carbon and energy source in a basal medium or a mixture of different substrates, consisting of sugars, organic acids, alcohols and/or hydrocarbons.

12. Method according to one of Claims 1 to 11, **characterized in that** the cultivation is carried out on a growth substrate or mixture before adding the tert-butyl compound or simultaneously with this for the induction of the TBA monooxygenase, where, following the induction, the conversion of the compound carrying the tertiary carbon atom to 2-methyl-1,2-dihydroxypropane takes place.

13. Method according to one of Claims 1 to 12, **characterized in that** the mixing ratio between growth substrate and substrate with tert-butyl compound is varied, where preferably a ratio of
[H₂] per mole of carbon to one molecule of the conversion substrate of between 1:1 to 1:0.01, particularly preferably between 1:0.5 to 1:0.1, is used, where [H₂] per mole of carbon corresponds to the reduction equivalent made available per mole of carbon of the growth substrate by the metabolism, and the quotient of [H₂] per mole of carbon is at least 1.

14. Method according to one of Claims 1 to 12, **characterized in that** ETBE is used as starting substrate, where the acetate released following cleavage of the ether bridges and its reduction equivalents released as a result of further metabolization are used for the oxidation of TBA to methyl-1,2-dihydroxypropane, or **in that** the further conversion of 2-methyl-1,2-dihydroxypropane is restricted and/or suppressed.

15. Method according to one of Claims 1 to 10, **characterized in that** an auxiliary substrate, preferably formic acid or salts thereof, is added for the regeneration of reduction equivalents.

## Revendications

1. Procédé de préparation enzymatique de 2-méthyl-1,2-dihydroxypropane, **caractérisé en ce qu'**on incube au moins un composé ayant un groupe tert-butyle dans une solution réactionnelle aqueuse qui comporte un système enzymatique et/ou produit ce dernier, qui comprend au moins l'une des séquences SEQ ID N°1 et SEQ ID N°2 et/ou leurs homologues à au moins 50 % et a une activité de monooxygénase, puis on obtient le 2-methyl-1,2-dihydroxypropane converti correspondant, le système enzymatique qui présente une activité de monooxygénase étant une enzyme isolée, un système enzymatique oligomère isolé et/ou un microorganisme ou un extrait brut de ce dernier, qui comprend ou produit le système enzymatique.

2. Procédé selon la revendication 1, **caractérisé en ce qu'**on utilise en tant que composés ayant un groupe tert-butyle le méthyl-tert-butyléther (MTBE), l'éthyl-tert-butyléther (ETBE), le tert-amyl-méthyléther (TAME), l'alcool tert-amylique (TAA), le tert-butoxyméthanol et/ou l'alcool tert-butylique (TBA).

3. Procédé selon la revendication 1 ou 2, **caractérisé en ce que** le système enzymatique ayant une activité de monooxygénase comprend la protéine ayant la séquence SEQ ID N°1 et la protéine ayant la séquence SEQ ID N°2, et/ou en combinaison avec chacun de ses homologues à au moins 50 %, la protéine SEQ ID N°1 représentant une phtalate dioxygénase et présentant une activité de monohydroxylase, et la protéine ayant la séquence SEQ ID N°2 ayant une activité de Fe/S-oxydoréductase, les homologues ayant les mêmes propriétés.

4. Procédé selon l'une des revendications 1 à 3, **caractérisé en ce qu'**on cultive des microorganismes qui contiennent un ou plusieurs gènes qui produisent une ou plusieurs enzymes ayant les séquences SEQ ID N°1 et 2 et/ou leurs homologues à au moins 50 % ayant une activité de monooxygénase, ou qui comprennent ces enzymes, ce à l'occasion de quoi ils provoquent dans des systèmes aqueux la conversion du ou des composés ayant un groupe tert-butyle en 2-méthyl-1,2-dihydroxypropane.

5. Procédé selon l'une des revendications 1 à 4, **caractérisé en ce qu'**on utilise un microorganisme qui code pour une protéine qui, en tant qu'enzyme oligomère constituée de deux sous-unités différentes, comprend les séquences d'acides aminés présentées dans SEQ ID N°1 et SEQ ID N°2 ou joue le rôle d'un hôte procaryote, dans lequel sont exprimés le gène ou les gènes qui présentent la séquence nucléotidique présentée dans SEQ ID N°3 et/ou SEQ ID N°4.

6. Procédé selon l'une des revendications 1 à 5, **caractérisé en ce qu'**on utilise du TBA en tant que substrat, ou on l'obtient ou le prépare à partir de l'un des autres composés de la revendication 2, ce à l'occasion de quoi un groupe méthyle du TBA est oxydé pour la conversion en le 2-méthyl-1,2-dihydroxypropane.

7. Procédé selon l'une des revendications 1 à 6, **caractérisé en ce que** le microorganisme est choisi parmi les souches bactériennes HCM-10 (DSM 18028), *Ideonella sp.* L108 (DSM 18260) ou *Methylibium sp.* R8 (DSM 19669).

8. Procédé selon l'une des revendications 1 à 7, **caractérisé en ce que** des cellules entières des microorganismes, non modifiées, subissent une perméabilisation ou sont utilisées en étant fixées à un support.

9. Procédé selon l'une des revendications 1 à 8, **caractérisé en ce qu'**on utilise des extraits cellulaires et/ou une monooxygénase ou un système de monooxygénases, après isolement partiel ou complet à partir des microorganismes, éventuellement sous forme purifiée, de préférence des extraits bruts sans cellules des microorganismes.

10. Procédé selon l'une des revendications 1 à 9, **caractérisé en ce que**, pour la culture, on utilise du TBA en tant que source unique de carbone et d'énergie dans un milieu de base.

11. Procédé selon l'une des revendications 1 à 10, **caractérisé en ce qu'**on ajoute pour la culture des substrats de croissance, de préférence un sucre, un acide organique, un alcool ou un hydrocarbure en tant que source unique ou supplémentaire de carbone ou d'énergie dans un milieu de base, ou un mélange de substrats différents constitués de sucres, d'acides organiques, d'alcools et/ou d'hydrocarbures.

12. Procédé selon l'une des revendications 1 à 11, **caractérisé en ce que** la culture est mise en oeuvre sur un substrat ou un mélange de croissance avant addition du composé tert-butylé ou simultanément à cette dernière, pour induction de la TBA monooxygénase, la conversion du composé portant l'atome de carbone tertiaire en 2-méthyl-1,2-dihydroxypropane ayant lieu après l'induction.

13. Procédé selon l'une des revendications 1 à 12, **caractérisé en ce qu'**on fait varier le rapport de mélange du substrat de croissance et du substrat comportant un composé tert-butylé, en utilisant de préférence un rapport entre [H₂] par mole de carbone et une molécule du substrat de conversion compris entre 1:1 et 1:0,01, d'une manière particulièrement préférée entre 1:0,5 et 1:0,1, le [H₂] par mole de carbone correspondant aux équivalents de réduction qui ont été rendus disponibles par mole de carbone du substrat de croissance grâce au métabolisme, le quotient [H₂] par mole de carbone étant d'au moins 1.

14. Procédé selon l'une des revendications 1 à 12, **caractérisé en ce qu'**on utilise en tant que substrat de départ de l'ETBE, ce pour quoi on utilise pour l'oxydation du TBA en méthyl-1,2-dihydroxypropane l'acétate libéré après dissociation du pont éther, et ses équivalents de réduction libérés par une métabolisation plus poussée, ou encore on restreint ou supprime la réaction plus poussée du 2-méthyl-1,2-dihydroxypropane.

15. Procédé selon l'une des revendications 1 à 10, **caractérisé en ce que**, pour la régénération d'équivalents de réduction, on ajoute un substrat auxiliaire, de préférence l'acide formique ou ses sels.
